# EUROPÄISCHE PATENTSCHRIFT

(11) **EP 1 691 793 B1**
(45) Veröffentlichungstag und Bekanntmachung des Hinweises auf die Patenterteilung: **03.09.2008**
(21) Anmeldenummer: 04803784.0
(22) Anmeldetag: 13.12.2004
(51) Int. Cl.: A61K 9/70

(54) **DARREICHUNGSFORM BASIEREND AUF VERNETZTEN HYDROPHILEN POLYMEREN**
ADMINISTRATION FORM BASED ON CROSS-LINKED HYDROPHILIC POLYMERS
FORME D'ADMINISTRATION A BASE DE POLYMERES HYDROPHILES RETICULES

(30) Priorität: 12.12.2003 DE 10358747
(43) Veröffentlichungstag der Anmeldung: 23.08.2006
(73) Patentinhaber: LTS LOHMANN Therapie-Systeme AG, 56626 Andernach (DE)
(72) Erfinder: BARTHOLOMÄUS, Johannes, 52080 Aachen (DE); VAZQUEZ LANTES, Maria Christina, 85635 Siegertsbrunn (DE)
(74) Vertreter: Schmidt, Werner
(86) Internationale Anmeldenummer: PCT/EP2004/014146
(87) Internationale Veröffentlichungsnummer: WO 2005/055991

(56) Entgegenhaltungen:
- DE-A1- 19 932 603
- ABLETSHAUSER C B ET AL: "Preparation and investigation of isolated MHPC-tannin films" PHARMAZIE 1992 GERMANY, Bd. 47, Nr. 11, 1992, Seiten 870-871, XP001205680 ISSN: 0031-7144
- ABLETSHAUSER C ET AL: "Self supporting polymer films crosslinked 'in situ' by simultaneous spraying of component solutions. I. Characterization and drug diffusion" FARMACEVTSKI VESTNIK 1994 SLOVENIA, Bd. 45, Nr. 4, 1994, Seiten 297-309, XP001205679 ISSN: 0014-8229
- OKUTGEN E ET AL: "Quantitative estimation of internal stress development in aqueous HPMC tablet film coats" INTERNATIONAL JOURNAL OF PHARMACEUTICS (AMSTERDAM), Bd. 119, Nr. 2, 1995, Seiten 193-202, XP002324919 ISSN: 0378-5173
- REMUNAN-LOPEZ C ET AL: "Mechanical and water vapor transmission properties of polysaccharide films" DRUG DEVELOPMENT AND INDUSTRIAL PHARMACY 1996 UNITED STATES, Bd. 22, Nr. 12, 1996, Seiten 1201-1209, XP009046354 ISSN: 0363-9045

## Beschreibung

Die vorliegende Erfindung betrifft eine filmförmige Darreichungsform zur oberflächlichen Verabreichung wenigstens eines Wirkstoffes und/oder Nährstoffes an ein Lebewesen umfassend wenigstens eine wirkstoffhaltige und/oder nährstoffhaltige Schicht basierend auf in-situ vernetzten hydrophilen Polymeren, die 30-60 Gew.-% Glycerin, bezogen auf die Gesamtmenge der vernetzten hydrophilen Polymere, als Weichmacher enthält und wobei die Darreichungsform eine Reiß festigkeit von mindestens 40N aufweist.

Filmförmige Darreichungsformen aus vernetzten hydrophilen Polymeren können zur oberflächlichen Verabreichung von Wirkstoffen und/oder Nährstoffen an ein Lebewesen eingesetzt werden, die in der wirkstoffhaltigen und/oder nährstoffhaltigen Schicht molekular oder partikulär verteilt vorliegen.

Entsprechende , filmförmige Darreichungsformen aus vernetzten hydrophilen Polymeren zur oberflächlichen Verabreichung an ein Lebewesen von Wirkstoffen und/oder Nährstoffen sind in der deutschen Offenlegungsschrift DE 199 32 603 A1 beschrieben. Obwohl solche filmförmige Darreichungsformen aus vernetzten hydrophilen Polymeren im feuchten Zustand gut verformbar sind, sind sie im trockenen Zustand je nach Schichtdicke mehr oder weniger starr. Diese geringe Plastizität im trockenen Zustand kann die oberflächliche Verabreichung wenigstens eines Wirkstoffes und/oder Nährstoffes an ein Lebewesen , wie zum Beispiel auf einer nasalen oder buccalen Schleimhaut des Menschen, erheblich erschweren.

Remuñán-lópez et al. (22(12), 1201-1209 1996) beschreibt die mechanischen Eigenschaften und die Wasserdampfdurchlässigkeit von verschiedenen Polysaccharid Filmen in Abhängigkeit vom Polymertyp, des Viskosität sowie Art und Menge an Weichmacher.

Es stellte sich daher die Aufgabe, eine filmförmige Darreichungsform zur oberflächlichen Verabreichung wenigstens eines Wirkstoffes und/oder Nährstoffes an ein Lebewesen aus einer Schicht basierend auf vernetzten hydrophilen Polymeren zur Verfügung zu stellen, die eine verbesserte Handhabung, insbesondere ein verbessertes Aufbringen der Darreichungsform auf der Oberfläche eines Lebewesens gewährleistet.

Diese Aufgabe wurde durch die Bereitstellung der erfindungsgemäßen filmförmigen Darreichungsform zur oberflächlichen Verabreichung wenigstens eines Wirkstoffes und/oder Nährstoffes an ein Lebewesen umfassend wenigstens eine wirkstoffhaltige und/oder nährstoffhaltige Schicht basierend auf in-situ vernetzten hydrophilen Polymeren, die 30-60 Gew.-%, bezogen auf die Gesamtmenge der vernetzten hydrophilen Polymere, Glycerin als Weichmacher enthält, und wobei die Darreichungsform eine Reißfestigkeit von mindestens 40N aufweist, gelöst.

Lebewesen im Sinne der beanspruchten Erfindung sind der Mensch, die Tiere und die Planzen, vorzugsweise der Mensch und die Tiere, besonders bevorzugt der Mensch.

Ganz besonders bevorzugt betrifft die beanspruchte Erfindung die transdermale oder transmukosale Verabreichung, insbesondere die transmukosale Verabreichung, wenigstens eines Wirkstoffes an Menschen.

Üblicherweise werden zur besseren Handhabung, d.h. insbesondere zur Erhöhung der Dehnbarkeit, Weichheit und Biegsamkeit, von relativ spröden Polymerfilmen Weichmachern in einer Menge bis zu 20 Gew. % bezogen auf die Polymermenge, eingesetzt.

Bei höheren prozentuallen Mengen an Weichmacher kann es zu Phasentrennungen z. B. durch Auskristallisieren kommen, so dass die Filme nicht mehr durchsichtig sind und ihre physikalischen Eigenschaften, wie die Reißfestigkeit, negativ beeinträchtigt werden. Beispielsweise führt ein Zusatz von 30 Gew. % Triethylcitrat, bezogen auf die Gesamtmenge eines vernetzten hydrophilen Polymers, zu weißen Filmen. Es kann sogar zum Ausscheiden des Weichmachers aus dem Film kommen.

Überraschenderweise gelingt es erfindungsgemäß, hohe Mengen an Glycerin in die wirkstoffhaltige und/oder nährstoffhaltige Schicht basierend auf in-situ vernetzten hydrophilen Polymeren einzuarbeiten und so die notwendige Verbesserung der Plastizität zu erreichen, ohne daß die Nachteile des Standes der Technik auftreten.

Für den Fachmann ist es selbstverständlich, daß die notwendige Menge Glycerin auch von der Dicke der jeweiligen Schicht aus vernetzten hydrophilen Polymeren abhängt. Generell liegt die erforderliche Menge im Bereich von 30 Gew.-% bis 60 Gew.% bezogen auf die Gesamtmenge vemetzter hydrophiler Polymere, wobei für dickere Schichten mehr Glycerin als für dünnere Schichten verwendet werden soll, um dieselbe Wirkung zu Wirkung zu erzielen.

Als hydrophile Polymere zur Herstellung der erfindungsgemäßen Darreichungsform werden vorzugsweise wasserlösliche Celluloseether, besonders bevorzugt Hydroxypropylmethylcellulose, Hydroxyethylcellulose und/oder Methylcellulose, ganz besonders bevorzugt Hydroxyrpropylmethylcellulose eingesetzt.

Diese in-situ Vernetzung der filmbildenden, auf hydrophilen Polymeren basierende Schicht erfolgt vorzugsweise während der Schichtbildung mit Hilfe von bekannten Vernetzern, vorzugsweise phenolischen Vemetzem und/oder Polyacrylsäurederivaten, besonders bevorzugt Tannin und/oder einer vernetzten, gegebenenfalls teilneutralisierten Polyacrylsäure (Polycarbophil®). Als geeignet hat sich ein Gewichtsverhältnis von hydrophilen Polymeren zu Vernetzer von 2:1 zu 5:1 herausgestellt und als besonders geeignet hat sich ein Gewichtsverhältnis von 4:1 herausgestellt. Durch die Vernetzung der filmbildenden hydrophilen Polymere ist es möglich, eine ausreichend sichere Handhabung der filmförmigen Darreichungsform, z. B. beim Herausnehmen aus der Verpackung und Aufbringen der Darreichungsform auf der Oberfläche eines Lebewesens , ohne Beschädigung der Darreichungsform durch Zerreißen zu gewährleisten. Durch die Vernetzung gelingt es erfindungsgemäß, filmförmige Darreichungsformen mit einer mindestens Reißfestigkeit von 40 N, vorzugsweise von mindestens 50 N, besonders bevorzugt von mindestens 60 N zur Verfügung zu stellen.

Die erfindungsgemäße filmförmige Darreichungsform wird zur oberflächlichen Verabreichung wenigstens eines Wirkstoffes und/oder Nährstoffes an ein Lebewesen eingesetzt.

Bezüglich der in der wirkstoffhaltigen und/oder nährstoffhaltigen Schicht enthaltenen Wirkstoffe und/oder Nährstoffe gibt es prinzipiell keine Einschränkung. Vorzugsweise sind die Wirkstoffe bzw. Nährstoffe jedoch Duftstoffe, Aromen, Diagnostica, Pflanzenschutzmittel, pharmazeutische Wirkstoffe, Vitamine, Düngemittel und/oder andere Nährstoffe.

Als pharmazeutischewirkstoffe können Analgetica, Antiallergica, Antibiotica, Antiemetica, Antiseptica, Antihistaminica, Antihypertonia, Appetitzügler, Cardiaca, Chemotherapeutica, Enzympräparate, Hormone, Immunmodulatoren, Impfungen, Lokalanästhetica, Psychopharmaka, Spasmolytica, Virustatica, Vitamine und Zytostatica verwendet werden.

Geeignete Wirkstoffe sind insbesondere Diamorphin, Alflentanil, Sufentanyl, Pentazocin, Buprenorphin, Nefopam, Flupirtin, Tramadol, Oxycodon, Metamizol, Propyphenanzon Phenazone, Nifenazon, Phenylbutazon, Oxyphenbutazon, Mofebutazon, Diflunisal, Meptazinol, Methadon, Pethidin, Meloxicam, Fenbufen, Mefenamisäure, Tenoxicam, Azapropazon, Piritramid, Tramadol, Amantadin, Benzotropin, Procyclidin, Moclobemid, Tranylcypromid, Maprotilin, Doxepin, Opipramol, Desipramin, Imipramin, Fluroxamin, Paroxetin, Trazodon, Viloxazin, Fluphenazin, Perphenazin, Promethazin, Thioridazin, Triflupromazin, Prothipendyl, Tiotixen, Chlorprothixen, Pipamperon, Pimozid, Fenethyllin, Trifluoperazin, Thioridazin, Oxazepam, Alprazolam, Clobazam, Piracetam, Melfalan, Cyclophosphamid, Trofosfamid, Chlorambucil, Lomustin, Busilfan, Prednimustin, Mercaptopurin, Thioguanin, Hydroxycarbamid, Altretamin, Procarbazin, Lisurid, Methysergid, Pizotifen, Roxatidin, Pirenzipin, Proglumid, Bromoprid, Pheniramin, Dimethinden, Tritoqualin, Loratadin, Doxylamin, Mequitazin, Dexchlorpheniramin, Triprolidin, Oxatomid, Moxonidin, Doxazosin, Urapidil, Dihydralazin, Deserpidin, Alprenolol, Bupranolol, Penbutolol, Esmolol, Ciliprolol, Metipranolol, Nadolol, Quinapril, Fosinopril, Cilazapril, Democlocyclin, Lymecyclin, Oxytetracyclin, Sulfamethopyrazin, Aerosoxacin, Becampicillin, Piperacillin, Pivampicillin, Cloxacillin, Flucloxacillin, Metronidazol, Clindamycin, Cefaclor, Cefpodoxim, Cephalexin, Cefradin, Pirbuterol, Orciprenalin, Clenbuterol, Procaterol, Cholintheophyllinat, Theophyllin-ethylenediamin, Ketofen, Viquidil, Procainamid, Mexiletin, Tocainid, Ipratropium, Tobutamid, Gliquidon, Gliborurid, Tolazamid, Acarbose und pharmazeutisch aktive Salze oder Ester der vorgenannten Wirkstoffe sowie Kombinationen von zwei oder mehreren dieser Wirkstoffe oder deren Salze oder Ester.

Geeignete Wirkstoffe sind beispielsweise Acebutolol, Acetylcystein, Acetylsalicylsäure, Aciclovir, Albrazolam, Alfacalcidol, Allantoin, Allopurinol, Ambroxiol, Amikacin, Amiloride, Aminoessigsäure, Amiodaron, Amitriptylin, Amlodipin, Amoxicillin, Ampicillin, Ascorbic Acid, Aspartam, Astemizole, Atenolol, Beclometason, Benserazid, Benzalkonium Hydrodrochlorid, Benzocain, Benzoesäure, Betametasone, Bezafibrate, Biotin, Biperiden, Bisoprolol, Bromacepam, Bromhexine, Bromocriptine, Budesonide, Bufexamac, Buflomedil, Buspirone, Coffein, Campher, Captopril, Carbamacipine, Carbidopa, Carboplatin, Cefaclor, Cefalexin, Cefadroxil, Cefazolin, Cefixime, Cefotaxim, Ceftazidin, Ceftriaxon, Cefuroxim, Celedilin, Chloramhenicol, Chlorhexidin, Chlorpheniramin, Chlortalidon, Cholin, Ciclosporin, Cilastatin, Cimetidin, Ciprofloxacin, Cisaprid, Cisplatin, Clarithromycin, Clavulansäure, Clomibramin, Clonazepam, Clonidin Clotrimazol, Codein, Colestyramin, Cromoglicinsäure, Cyanocobalamin, Cyproteron, Desogetrel, Dexamethason, Dexpanthenol, Dexthromethorphan, Dextropropoxiphen, Diazepam, Diclofenac, Digoxin, Dihydrocodein, Dihyderoergotamin, Dihydroergotoxin, Diltiazem, Diphenhydramin, Dipyridamol, Dipyron, Disopyramid, Domperidon, Dopamin, Doxcyclin, Enalapril, Ephedrin, Epinephrine, Ergocalciferol, Ergotamin, Erytrhomycin, Estradiol, Ethinylestradinol, Etoposid, Famotidin, Felodipin, Fenofibrat, Fenoterol, Fentanyl, Flavin Mononucleotid, Fluconazol, Flunarizin, Fluorouracil, Fluoxetin, Flurbiprofen, Furosemid, Gallopamil, Gemfibrozil, Gentaminicin, Ginkgo Biloba, Glibenclamid, Glipizid, Glozapin, Glycyrrhiza Glabra, Griseofulvin, Guaifenesin, Haloperidol, Heparin, Hyaluronsäure, Hydrochlorothiazid, Hydrocodon, Hydrocortison, Hydromorphon, Ibratropium Hydroxide, Ibuprofen, Imipenem, Indomethacin, lohexol, Iopamidol, Isosorbid Dinitrat, Isosorbid Mononitrat, Isotretionin, Ketotifen, Ketoconazol, Ketoprofen, Ketorolac, Labatalon, Lactulose, Lecithin, Levocamitin, Levodopa, Levoglutamid, Levonorgestrel, Levothyroxin, Lidocain, Lipase, Lipramin, Lisinopril, Loperamid, Lorazepam, Lovastatin, Medroxyprogesteron, Menthol, Methotrexat, Methyldopa, Methylprednisolon, Metoclopramid, Metoprolol, Miconazol, Midazolam, Minocyclin, Monoxidil, Misoprostol, Morphin, Multivitamine und Mineralien, N-Methylephedim, Naftidrofuril, Naproxen, Neomycin, Nicardipin, Nicergolin, Nicotinamid, Nicotin, Nicotinsäure, Nifedipin, Nimodipin, Nitrazepam, Nitrendipin, Nizatidin, Norethisteron, Norfloxacin, Norgestrel, Nortriptylin, Nystatin, Ofloxacin, Omeprazol, Ondansetron, Pancreatin, Panthenol, Pantothensäure, Paracetamol, Penicillin G, Penicillin V, Phenobarbital, Phenoxifyllin, Phenoxymethylpenicillin, Phenylephrin, Phenylpropanolamin, Phenytoin Piroxicam, Polymxyxin B, Povidone-lod, Pravastatin, Prazepam, Prazosin, Prednisolon, Prednison, Propafenon, Propranolol, Proxyphyllin, Pseudoephedrin, Pyridoxine, Chinidin, Ramipril, Ranitidin, Reserpin, Retinol, Riboflavin, Rifampicin, Rutosid, Saccharin, Salbutamol, Salcatonin, Salicylsäure, Simvastatin, Somatropin, Sotalol, Spironolacton, Sucralfat, Sulbactam, Sulfamethoxazol, Sulfasalazin, Sulpirid, Tamoxifen, Tegafur, Teprenon, Terazosin, Terbutalin, Terfenadin, Tetracyclin, Theophyllin, Thiamin, Ticlopidin, Timolol, Tranexamsäure, Tretinoin, Triamcinolone Acetonide, Triamteren, Trimethoprim, Troxerutin, Uracil, Valproinsäure, Vancomycin, Verapamil, Vitamine E, Zidovudine.

Weiterhin geeignete Wirkstoffe sind Proclorperazin-edisylal, Eisen-II-sulfat, Aminocapronsäure, Kaliumchlorid, Mecamylamin-hydrochlorid, Procainamidhydrochlorid, Amphetamin-sulfat, Benzphetamin-hydrochlorid, Isoporterenol-sulfat, Methamphetamin-hydrochlorid, Phenmetrazin-hydrochlorid, Bethanecholchlorid, Methacholin-chlorid, Pilocarpin-hydrochlorid, Atropinsulfat, Methascopolamin-bromid, Isopropamid-iodid, Tridihexethylchlorid, Phenformin-hydrochlorid, Methylphenidathydrochlorid, Oxprenolol-hydrochlorid, Metroprolol-tartrat, Cimetidin-hydrochlorid, Diphenidol, Meclizin-hydrochlorid, Proclorperazinmaleat, Phenoxybenzamin, Thiethylperazin-maleat, Anisindon, Diphenadion, Erythrit-tetranitrat, Dizoxin, Isofurophat, Acetazolamid, Methazolamid, Bendroflumethiazid, Chlorpropamid, Tolazamid, Chlormadinon-acetat, Phenaglycodol, Aluminium-aspirin, Methotrexat, Acetyl-sulfioxazol, , Progestine, österrogene Steroide, progestatine Steroide, Corticosteroide, 17-β-östradiol, Ethinyl-östradiol-3-methyl-ester, Hydrocorticosteronacetat, Methyltesteron, 17-α-Hydroxyprogesteron-acetat, 19-Nor-progesteron, Norethindron, Progesteron, Norgesteron, Norethynodrel u. a.

Weitere Beispiele von Wirkstoffen sind Fenoprofen, Sulindac, Indoprofen, Nitroglycerin, Timolol, Alprenolol, Imipramin, Chlorpromazin, Dihydroxyphenylalanin, Pivaloxyloxyethylester von α-Methyldopa-hydrochlorid, Calcium-gluconat, Eisen-IIlactat, Vincamin, Phenoxybenzamin, Blocker u. ä. Die Wirkstoffe sind bekannt aus "Pharmaceutical Sciences" von Remington, 14. Auflage, 1979, Mack Publishing Co., Easton, Pennsylvania; "The Drug, The Nurse, The Patient, Including Current Drug Handbook", 1974-1976, von Falconer et al, Saunder Co., Philadelphia, Pennsylvania, und "Medical Chemistry", 3. Auflage, Band 1 und 2, von Burger, Wiley-Interscience, New York.

Repräsentative Arzneimittel, die an warmblütige Tiere, beispielsweise Wiederkäuer, mit Hilfe der erfindungsgemäßen Darreichungsform verabreicht werden können, sind u. a. Anthelmintica, wie Mebendazol, Levamisol, Albendazol, Cambendazol, Fenbendazol, Parbendazol, Oxfendazol, Oxybendazol, Thiabendazol, Tichlorfon, Praziquantel, Morantel und Pirantel, u. ä.; Antiparasiten-Mittel, wie Avermectine und Ivermectin, wie in den US-PS 41 99 569 und 43 89 397 (Merck) und in "Science", Band 221, S. 823 - 828, 1983 angegeben, wo diese Ivermectin-Antiparasiten-Mittel als geeignet zur Unterstützung bei der Bekämpfung von üblicherweise bei Säugetieren auftretenden Würmern, wie Rundwürmern (Spulwürmem), Lungenwürmem u. ä., angegeben sind, und auch daß das Ivermectin geeignet ist zur Behandlung von Insekteninfektionen, wie Maden, Läusen, Milbenräude u. ä.; antimikrobielle Mittel, wie Chlortetracyclin, Oxytetracyclin, Tetracyclin, Gentamicin, Streptomycin, Dihydrostreptomycin, Bacitracine, Erthromycin, Ampicilline, Penicilline, Cephalosporine u. ä.; Schwefel enthaltende Arzneimittel (sufa drugs), wie Sulfamethazin, Sulfathiazol u. ä.; Wachstumsstimulantien, wie Monesin®-natrium und Elfazepam®; Anti-Flohmittel (defleaing agents), wie Dexamethazon und Flumethazon; die Verdauung im Pansen beeinflussende Mittel und lonophore, wie Lasalocid, Virginamiycin, Salinomycin und Ronnel; Mineralstoffe, wie Kupferoxid, Cobaltsulfat, Kaliumiodat, Zinkoxid, Mangansulfat, Zinksulfat, Selen, Natriumselenit, günstige Mineralsalze u. ä.; Antiblähmittel, wie organische Polysiloxane; hormonelle Wachstumszusätze, wie Stilböstrol; Vitamine, wie Vitamine A und D; mit 500 000 : 100 000 IU/f, Vitamin E mit 500 000 IU/f u. ä.; Antienteritismittel, wie Furazolidon, Wachstumsfaktoren, Nährstoffzusätze, wie Lysinmonohydrochlorid, Methionin, Magnesiumcarbonat u. ä.; β-Agonisten, Elenbuterol u. ä., und chemische Markierungsstoffe, wie Chromoxid, und Salze von Ytterbium und Erbium.

Die lokal wirkenden Wirkstoffe umfassen weiterhin Fungizide wie Amphotericin B, Antibiotika, wie Penicilline, Cephalosporine, Erythromycin, Tetracyclin, Aminoglycoside, antivirale Verbindungen wie Acyclovir, Idoxuridin, Atemverbesser wie Chlorophyll, Gewebewachstums hemmende Verbindungen, Antikariesverbindungen wie Metallfluoride, insbesondere Natriummonofluorphosphat, Zinnfluorid, Aminfluorid, Schmerzmittel wie Methylsalicylat, Lokalanästhetika wie Benzocain, orale Antiseptika wie Chlorhexidin und dessen Salze, Hexylresorcin, Dequalinium Chlorid, Cetylpyridin Chlorid), Antientzündungsmittel, Hormone wie Oestriol, Antiplaqueverbindungen wie Chlorhexidin und dessen Salze, Octenidin, oder Mischungen von Thymol, Menthol, Methysalicylat, Eucalyptol, Pufferverbindungen wie Kaliumphosphat, Calciumcarbonat, Natriumbicarbonat, Natrium- und Kaliumhydroxid sowie Desensibilisatoren für Zähne wie z. B. Kaliumnitrat.

Desweiteren sind als aktive Wirkstoffe Desinfektiva wie Chlorverbindungen, insbesondere Calciumhypochlorit, ein Insektizid, Pestizid, Herbizid, Fungizid, oder Wachstumförderer bzw. Düngemittel wie z. B. Stickstoff haltige Verbindungen, insbesondere Harnstoff, Harnstofformaldehydverbindungen, Caliumnitrat, Caliumsulfat, Caliumchlorid, Ammoniumnitrat, Ammoniumsuflat, Monoammoniumphosphat, dibasisches Ammoniumphosphat, Ammoniumphosphorsäureverbindungen, Spurenelemente für Nahrungsmittel wie Eisen, Zink, Mangan, Kupfer, Bor, Molybden oder Mischungen daraus geeignet.

Wirkstoffe, die sich für die erfindungsgemäße Darreichungsform eignen, sind auch Steroidhormone wie:
Gestagen wirksame Steroidhormone, wie beispielsweise das 13-Ethyl-17β-hydroxy-18,19-dinor-17α-pregn-4-en-20yl-3-on, das 13-Ethyl-17β-hydroxy-18,19-dinor-17α-pregna-4,15-dien-20yn-3-on (=Gestoden), das 13-Ethyl-17β-hydroxy-11-methylen-18,19-dinor-17α-pregn-4-en-20yn oder das 13-Ethyl-11-methylen-17β-hydroxy-18,19-dinor-17α-pregn-4-en-3-on (3-Keto-Desogestrel) Estrogen wirksame Steroidhormone das 3-Hydroxy-1,3,5-(10)-estratrien-17-on (=Estron), das 1,3,5(10)-Estratrien-3,17β-diol oder das 1,9-Nor-17α-pregna-1,3,5(10)-trien-20yn-3,17β-diol, das 17β-Hydroxy-19-nor-17a-pregn-4en-20yn-3-on, das 14α, 17α-Ethano-1,3,5(10)-estratrien-3,17β-diol (=Cyclodiol) und das 14α, 17α-Ethano-1,3,5(10)-estratrien-3,16α,17β-triol (=Cyclotriol) und Kombinationen dieser Gestagene und Estrogene.
Androgen wirksame Steroidhormone wie das 17β-Hydroxy-4-androsten-3-on (=Testosteron) und dessen Ester oder das 17β-Hydroxy-1α-methyl-5α-androsten-3-on (=Mesterolon).
Antiandrogen wirksame Steroidhormone wie das 17α-Acetoxy-6-chlor-1β,2β-dihydro-3H-cyclopropa[1,2]-pregna-1,4,6-trien-3,20-dion.
Kortikoide wie das 11β,17α,21-Trihydroxy-4-pregnen-3,20-dion, das 11β,17α,21-Trihydroxy-1,4-pegnadien-3,20-dion, das 11β,17α,21-Trihydroxy-6α-methyl-1,4-pregnatrien-3,20-dion und das 6α-Fluor-11β,21-dihydroxy-16α-methyl-1,4-pregnadien-3,20-dion (=Diflucortolon) und deren Ester.

Geeignete Wirkstoffe sind ferner:
Ergolin-Derivate, wie das Lisurid, [3-(9,10-Didehydro-6-methyl-8a-ergolinyl)-1,1-diethylharnstoff], das Bromlisurid [=3-(2-Brom-9,10-dehydro-6-methyl-8α-ergolinyl-1,1-diethylharnstoff], das Tergurid [=3-(6-Methyl-8α-ergolinyl-1,1-diethylharnstoff] und das Protergurid [=3-(6-Propyl-8α-ergolinyl)-1,1-diethylharnstoff].
Antihypertonika wie das 7α-Acetylthio-17α-hydroxy-3-oxo-4-pregnen-21-carbonsäure-γ-lacton und das 7α-Acetylthio-15β-,16β-methylen-3-oxo-17α-pregna-1,4-dien-21,17-carbolacton (=Mespirenon).
Antikoagulantia wie die 5-[Hexahydro-5-hydroxy-4-(3-hydroxy-4-methyl-1-octen-6-ynyl)-2(1 H)-pentalenyliden)]-pentansäure (=Iloprost) oder die (Z)-7-[(1R,2R,3R,5R)-5-Chlor-3-hydroxy-2-[(E)-(3R)-3-hydroxy-4,4-dimethyl-1-octenyl]-cyclopentyl]-5-heptensäure (=Nocloprost).
Psychopharmaka wie das 4-(3-Cyclopentyloxy-4-methoxy-phenyl-2-pyrrolidon (=Rolipram) und das 7-Chlor-1,3-dihydro-1-methyl-5-phenyl-2H-1,4-benzodiazepin-2-on.

Bei erfindungsgemäßen Darreichungsformen bestehend aus Schichten basierend auf vernetzten hydrophilen Polymeren, insbesondere mit hohen Mengen an Glycerin (> 40 Gew. %), besteht die Gefahr eines mikrobiellen Zerfalls, wenn sie über eine längere Zeit unter unsterilen Bedingungen gelagert werden. In diesem Fall sind Konservierungsmittel erforderlich. Als Konservierungsmittel eignen sich alle üblichen Konservierungsmittel. Es können Konservierungsmittel unter anderem aus der Gruppe von Alkoholen (z.B. Chlorbutanol, Phenylethylalkohol oder Benzylalkohol), Säuren (z.B. Sorbinsäure, Benzoesäure oder Borsäure), PHB-Ester (z.B. Parabene), Phenolderivate (z.B. Phenol, Kresol oder Chlorkresol), Quartäre Verbindungen bzw. Quaternäre Ammoniumverbindungen (Quats) (z.B. Benzalkoniumchlorid), Organo-Hg-Verbindungen (z.B. Thiomersal, Phenylmercurinitrat oder Phenylmercuriborat) und Guanide (z.B. Chlorhexidin oder Chlorhexidinacetat) sein. Es können auch Mischungen von mindestens zwei Konservierungsmittel eingesetzt werden.

Die erfindungsgemäßen filmförmigen Darreichungsformen können ein- oder mehrschichtig sein. Sofern die filmförmigen Darreichungsformen mehrschichtig sind, können sie mehr als eine wirkstoffhaltige und/oder nährstoffhaltige Schicht und eine Haftschicht und/ oder eine Deckschicht aufweisen.

In der erfindungsgemäßen filmförmigen Darreichungsform basiert/basieren die wirkstoffhaltigen und/oder nährstoffhaltigen Schicht(en) auf vernetzten hydrophilen Polymeren und enthält/enthalten Glycerin. Die wirkstoffhaltigen und/oder nährstoffhaltige(n) Schicht(en) kann/können den Wirkstoff in einer molekulären und/oder partikulären Form enthalten.

Aus der wirkstoffhaltigen und/oder nährstoffhaltigen Schicht bzw. den weiteren vorhandenen wirkstoffhaltigen und/oder nährstoffhaltigen Schichten kann die Wirkstoff- und/oder die Nährstoff-Freisetzung nicht nur über die unterschiedliche Wirkstoff- und/oder Nährstoff-Konzentration, sondern auch über den Grad der Vernetzung der hydrophilen Polymere gesteuert werden. Innerhalb einer wirkstoffhaltigen und/oder nährstoffhaltigen Schicht kann beispielsweise über einen Konzentration-Gradienten des Wirkstoffs und/oder des Nährstoffs die Freisetzung gesteuert werden. Eine weitere Möglichkeit, die Wirkstoff- und/oder die Nährstoff-Freisetzung zu beeinflussen, besteht darin, mehrere wirkstoffhaltige und/oder nährstoffhaltige Schichten mit unterschiedlichen Wirkstoff- und/oder Nährstoff-Konzentrationen in den erfindungsgemäßen filmförmigen Darreichungsformen vorzusehen. Weiterhin können auch wirkstofffreie bzw. nährstofffreie Schichten, ggf. aus vernetzten hydrophilen Polymeren, zwischen den wirkstoffhaltigen bzw. nährstoffhaltigen Schichten vorliegen. So kann damit aus einer ersten wirkstoffhaltigen auf hydrophilen Polymeren basierenden Schicht der Wirkstoff schnell und in einer ausreichenden Menge zur Erzielung einer unmittelbaren Wirkung freigesetzt werden, während aus weiteren wirkstoffhaltigen Schichten eine länger andauernde Wirkstoff-Freisetzung zur Erzielung einer anhaltenden Wirkung ermöglicht wird.

Die wirkstoffhaltige und/oder nährstoffhaltige Schicht weist vorzugsweise eine Dicke von 30-500 µm auf.

Um eine ausreichende Haftung der erfindungsgemäßen Darreichungsform bei transmukosaler bzw. transdermaler Applikation zu gewährleisten, kann entweder ein bioadhäsives Polymer in die wirkstoffhaltige und/oder nährstoffhaltige Schicht eingearbeitet werden oder eine zusätzliche Schicht als Haftschicht in der erfindungsgemäßen Darreichungsform vorgesehen werden. Eine Haftschicht kann aus einem oder mehreren der bekannten bioadhäsiven Polymeren, wie z. B. Polyacrylsäurederivaten, bestehen. Beispielsweise kann die Haftschicht aus einer Mischung aus gegebenenfalls vernetzten hydrophilen Polymeren und einem Polyacrylsäurederivat oder nur aus Polyacrylsäurederivaten bestehen. Als bioadhäsive Polyacrylsäurederivate eignen sich Polyacrylsäuren, die ggf. zum Teil als Calcium Salz vorliegen und ggf. vernetzt sind. Besonders bevorzugt sind zum Teil als Calcium Salz vorliegende Polyacrylsäuren vernetzt mit Divinylglycol. Solche Produkte sind als Polycarbophile® marktgeführt.

Die Haftschicht kann aus einer Mischung aus einem oder mehreren der genannten bioadhäsiven Polymeren, wie z. B. Ethylcellulose, bestehen, insbesondere wenn eine zusätzliche Steuerung der Wirkstofffreisetzung mit Hilfe der Haftschicht erwünscht ist.

Die Haftschicht weist vorzugsweise eine Dicke von 10 bis 100 µm auf.

Die erfindungsgemäße filmförmige Darreichungsform weist vorzugsweise auch eine Deckschicht auf. Die Deckschicht besteht vorzugsweise aus einem wasserunlöslichen Polymer und ist für den Wirkstoff und/oder Nährstoff undurchlässig. Damit wird eine unidirektionale Wirkstofffreisetzung und/oder Nährstofffreisetzung gewährleistet. Bei dieser unidirektionalen Freisetzung wird der Wirkstoff und/oder der Nährstoff nur an den Applikationsort abgegeben.

Die Deckschicht kann aus vernetzten hydrophilen Polymeren, beispielsweise aus mit Tannin vernetzten Hydroxypropylmethylcellulose, aufgebaut sein.

Weiterhin ist es möglich, die Deckschicht aus wenigstens einem wasserunlöslichen Celluloseether, vorzugsweise aus Alkylcellulose, besonders bevorzugt aus Ethylcellulose, oder einem Celluloseester, vorzugsweise Celluloseacetat, und/oder einem wasserunlöslichen Poly(meth)acrylat, vorzugsweise einem Poly(C1-4)-alkyl(meth)acrylat, Poly(C1-4)-dialkylamino-(C1-4)alkyl(meth)acrylat und/oder deren Copolymere, ganz besonders bevorzugt einem Copolymer aus Ethylacrylat/ Methylmethacrylat und/ oder einem Copolymer aus Ethylacrylat/Methylmethacrylat/Trimethylammoniumethylmethacrylat-Chlorid aufzubauen. Gegebenenfalls können die Celluloseether, die Celluloseester und die Poly(meth)acrylate Weichmacher enthalten.

In einer bevorzugten Ausführungsform der beanspruchten Erfindung ist die Deckschicht aus Ethylcellulose oder aus einem Copolymer aus Ethylacrylat/Methylmethacrylat/Trimethylammoniumethylmethacrylat-Chlorid mit einem molaren Verhältnis der jeweiligen Monomeren von 1 : 2 : 0,1, in beiden Fällen mit einer prozentualen Menge am Weichmacher, bevorzugt Triethylcitrate, von 20 bis 40 Gew. % bezogen auf die Menge an Polymer. Ganz besonders bevorzugt ist eine Deckschicht bestehend aus einem Copolymer aus Ethylacrylat/Methylmethacrylat mit einem molaren Verhältnis der jeweiligen Monomeren von 2 : 1 (Weichmacher Zusatz in diesem Fall ist nicht unbedingt erforderlich).

Die Deckschicht weist vorzugsweise eine Dicke von 10 bis 100 µm auf.

Die erfindungsgemäße filmförmige Darreichungsform kann vor der Applikation mit einer Schutzschicht bedeckt sein.

Die erfindungsgemäße filmförmige Darreichungsform wird hergestellt, indem die wirkstoffhaltige und/oder nährstoffhaltige Schicht bzw. die wirkstoffhaltigen und/oder nährstoffhaltigen Schichten, vorzugsweise aus einer wässrigen Lösung der hydrophilen Polymere, die Glycerin enthält, und des Wirkstoffs durch Auftragen unter gleichzeitiger oder nachträglicher Einwirkung des Vernetzers, vorzugsweise als wässrige Lösung, und Entfernung des Wassers durch Trocknen gebildet wird.

Auf die getrocknete wirkstoffhaltige und/oder nährstoffhaltige Schicht kann durch Aufbringen einer wässrigen Dispersion wie einer Latex bzw. Pseudolatex-Dispersion eines wasserunlöslichen Polymeren oder einer Lösung eines solchen Polymeren in einem geeigneten, organischen Lösungsmittel unter anschließender Entfernung des Wassers oder organischer Lösungsmittel durch Trocknen und/ oder Vakuumbehandlung die Deckschicht hergestellt werden.

Sofern bei der erfindungsgemäßen filmförmigen Darreichungsform eine Haftschicht vorhanden ist, wird diese vorzugsweise aus einer wässrigen Dispersion von Polyacrylsäuren, die ggf. zum Teil als Calcium Salz vorliegen und ggf. vernetzt sind.

Vorzugsweise wird die erfindungsgemäße filmförmige Darreichungsform hergestellt, indem die einzelnen Schichten übereinander auf einer glatten Oberfläche aufgebaut werden, wobei das jeweils filmbildende Polymer zusammen mit dem ggf. vorhandenen Vernetzer, dem ggf. vorhandenen Glycerin und dem ggf. vorhandenen Wirkstoff pro Schicht jeweils durch Versprühen und Trocknen als Teilschichten aufgebracht wird. Die Trocknung erfolgt dabei vorzugsweise simultan mit dem Versprühen. Die Teilschichten haben vorzugsweise eine Dicke von 0,1 bis 10 µm.

Das Versprühen der wäßrigen Lösung von hydrophilen Polymeren und der wäßrigen Lösung des Vernetzers erfolgt vorzugsweise gleichzeitig, wobei sich die hydrophilen Polymere und der Vernetzer nach dem Versprühen vermischen und danach in situ das Polymer vernetzt wird.

Sofern in einer Schicht der Wirkstoff und/oder Nährstoff vorhanden ist, erfolgt die Beladung vorzugsweise dadurch, dass der Wirkstoff und/oder Nährstoff in der wässrigen Lösung von hydrophilen Polymeren bereits gelöst ist, bevor diese Lösung mit der Lösung des Vernetzers zusammengebracht wird.

Die große Variabilität dieser Verfahrensweise erlaubt es, den Schichtaufbau in beliebiger Reihenfolge durchzuführen. So kann zuerst die Haftschicht oder zuerst die Deckschicht als Grundlage für die darauffolgenden Schichten gebildet werden.

Vorzugsweise wird zur Durchführung des Herstellungsverfahrens eine Apparatur wie in DE 101 46 251 beschrieben eingesetzt. Die entsprechende Offenbarung gilt als Teil der vorliegenden Offenbarung.

Diese Vorrichtung umfaßt mindestens eine Sprühvorrichtung, einen Trockner und mindestens eine Platte, die zyklisch unter der Sprühvorrichtung hindurch bewegt wird. Vorzugsweise weist die Vorrichtung mehrere Düsen auf, deren Sprühkegel sich überlappen.

### Bestimmung der Plastizität durch Zugversuch

Zur Ermittlung der mechanischen Eigenschaften wurden Zugversuche durchgeführt, wobei die maximal erreichbare Dehnung als Maß für die Plastizität der Darreichungsform eines Werkstoffes dient.

Zur Bestimmung der maximalen Dehnung und der Reißfestigkeit wird ein Texture Analyser TA.XT2i der Firma Winopal (Deutschland) eingesetzt. Filmstücke der wirkstoffhaltigen und/oder nährstoffhaltigen Schicht mit einer Länge von 9,5 cm und einer Breite von 1 cm werden mit Einspannbacken an beiden Enden eingeklemmt und leicht eingespannt, so dass die freie Spannlänge 7 cm beträgt. Die Einspannbacken sind mit Beschichtungen auf der Oberfläche, die mit den Stücken in Kontakt kommen, versehen, um ein vorzeitiges Zerreißen der Stücke an den Klammern zu vermeiden. Falls ein Stück trotz Beschichtungen an den Klammern zerreißen sollte, werden diese Werte nicht berücksichtigt. Mit einer konstanten Geschwindigkeit von 0,5 mm/s zieht die obere Klammer aufwärts. Die dabei zu jeder Zeitpunkt eingesetzte Kraft sowie die resultierende Dehnung wird von dem Texture Analyser aufgenommen. Die Kraft, die Dehnung und die Zeit werden dann mit der Hilfe einer Software dargestellt und analysiert.

Die Reißfestigkeit eines untersuchten Filmstückes ist die Kraft, die in dem Moment, in dem das jeweilige Stück zerreißt, gerade auf das Filmstück einwirkt.

Die maximale Dehnung ist das Ausmaß der Dehnung in dem Moment, in dem der jeweilige Stück zerreißt.

### Figuren

**Figur 1**
   Darstellung der eingesetzten Kraft gegen die resultierende Dehnung einer Schicht basierend auf vernetzter Hydroxyproplymethylcellulose ohne Weichmacherzugabe (Vergleichsbeispiel 1) bzw. 25 Gew.-%, bezogen auf die Gesamtmenge vernetzter Hydroxypropylmethylcellulose, Polyethylenglycol (Vergleichsbeispiel 2) bzw. Sorbitol (Vergleichsbeispiel 3) bzw. Glycerin (Beispiel 5). Die Filme mit Glycerin zeigen eine größere maximale Dehnung und damit eine höhere Plastizität als die Filme mit Polyethylenglycol bzw. Sorbitol.
**Figur 2**
   Darstellung der eingesetzten Kraft gegen die resultierende Dehnung einer Schicht basierend auf vemetzter Hydroxypropylmethylcellulose mit 20 Gew.-% (Beispiel 6) bzw. 50 Gew.-% (Beispiel 7), bezogen auf die Gesamtmenge vemetzter Hydroxypropylmethylcellulose, Glycerin. Je größer die Menge an Glycerin, desto größer ist die maximal erreichbare Dehnung und desto plastischer sind die Schichten. Filme mit 50% Glycerin zeigen eine Reißfestigkeit von über 60 N.
**Figur 3**
   Darstellung der eingesetzten Kraft gegen die resultierende Dehnung einer Schicht basierend auf vernetzter Hydroxypropylmethylcellulose mit 20 Gew.-%, bezogen auf die Gesamtmenge vemetzter Hydroxypropylmethylcellulose, Glycerin (Beispiel 6) bzw. Triethylcitrat (Vergleichsbeispiel 4). Die Filme mit Glycerin zeigen eine größere maximal erreichbare Dehnung und dabei eine höhere Plastizität als die Filme mit Triethylcitrat.

### Beispiele:

### Beispiel 1

Zur Herstellung der filmförmigen Darreichungsform wurde eine Lösung von 10 g Hydroxypropylmethylcellulose, 5 g Glycerin, 1 g des Wirkstoffes Prednisolon, und 484 g Wasser sowie eine Lösung aus 2,5 g Tannin in 497,5 g Wasser hergestellt. Mit Hilfe der in DE 101 46 251 beschriebenen Apparatur wurden diese beiden Lösungen jeweils mit einer Düse gleichzeitig auf eine Glasplatte aufgesprüht, bei 80 °C getrocknet und der Sprühvorgang nach Bildung der jeweiligen Teilschicht mehrmals wiederholt, bis eine Schichtdicke von 80 µm erreicht worden war.
Die auf diese Weise hergestellte Darreichungsform war leicht zu handhaben und leicht auf die menschliche Haut sowie auf die menschlichen Schleimhäute, zum Beispiel auf die buccale Schleimhaut, aufzubringen.

### Beispiel 2

a) Zur Herstellung der wirkstoffhaltige Schicht wurde eine Lösung von 10 g Hydroxypropylmethylcellulose, 7,5 g Glycerin, 1 g des Wirkstoffes Prednisolon, und 481,5 g Wasser sowie eine Lösung aus 2,5 g Tannin in 497,5 g Wasser hergestellt. Mit Hilfe der in DE 101 46 251 beschriebenen Apparatur wurden diese beiden Lösungen jeweils mit einer Düse gleichzeitig auf eine Glasplatte aufgesprüht, bei 80 °C getrocknet und der Sprühvorgang nach Bildung der jeweiligen Teilschicht mehrmals wiederholt bis eine Schichtdicke der wirkstoffhaltigen Schicht von 200 µm erreicht worden war.
b) Es wurde eine Dispersion aus 6 g Polyacrylsäure vernetzt mit Divinylglycol (Polycarbophil®) in 494 g Wasser hergestellt. Auch diese Dispersion wurde mit Hilfe der vorstehend angegebenen Apparatur in einem mehrmaligen Sprühvorgang auf der wirkstoffhaltigen Schicht in Teilschichten aufgetragen, bis eine Schichtdicke der Haftschicht von 50 µm erreicht worden war.
Die auf diese Weise hergestellte Darreichungsform war leicht zu handhaben und leicht auf die menschliche Haut sowie auf die menschlichen Schleimhäute, zum Beispiel auf die buccale Schleimhaut, aufzubringen.

### Beispiel 3

a) Zur Herstellung der Deckschicht wurde eine Lösung von 10 g Hydroxypropylmethylcellulose, 6,25 g Glycerin und 483,75 g Wasser sowie eine Lösung aus 2,5 g Tannin in 497,5 g Wasser hergestellt. Mit Hilfe der in DE 101 46 251 beschriebenen Apparatur wurden diese beiden Lösungen jeweils mit einer Düse gleichzeitig auf eine Glasplatte aufgesprüht, bei 80 °C getrocknet und der Sprühvorgang nach Bildung der jeweiligen Teilschicht mehrmals wiederholt bis eine Schichtdicke der Deckschicht von 50 µm erreicht worden war.
b) In der selben Art und Weise wie in a) beschrieben, wurde eine Lösung von 10 g Hydroxypropylmethylcellulose, 6,25 g Glycerin, 2 g Prednisolon, als Beispiel für den Wirkstoff, und 481,75 g Wasser, sowie eine Lösung von 2,5 g Tannin in 497,5 g Wasser mit Hilfe derselben Apparatur durch mehrmaligen Sprühvorgang auf der Deckschicht in Teilschichten aufgetragen bis eine Schichtdicke der wirkstoffhaltigen Schicht von 100 µm erreicht worden war.
Die auf diese Weise hergestellte Darreichungsform war leicht zu handhaben und leicht auf die menschliche Haut sowie auf die menschlichen Schleimhäute, zum Beispiel auf die buccale Schleimhaut, aufzubringen.

### Beispiel 4

a) Zur Herstellung der Deckschicht wurde ein durch Verdünnung von 333,33 g eines 30 %-igen wässrigen Latex mit 666,67 g Wasser erhaltenes 10 %-iges wässriges Latex aus einem Copolymer Ethylacryl/Methylmethacrylat mit einem molaren Verhältnis der Monomeren von 2:1, eingesetzt. Diese Dispersion wurde mit Hilfe der in DE 101 46 251 beschriebenen Apparatur in einem mehrmaligen Sprühvorgang, bei dem jeweils die Teilschichten erzeugt wurden, bis eine Schichtdicke der Deckschicht von 50 µm erreicht worden war.
b) Zur Herstellung der wirkstoffhaltige Schicht wurde eine Lösung von 10 g Hydroxypropylmethylcellulose, 7,5 g Glycerin, 2 g Prednisolon, als Beispiel für den Wirkstoff, und 480,5 g Wasser, sowie eine Lösung von 2,5 g Tannin in 497,5 g Wasser mit Hilfe derselben Apparatur durch mehrmalige Sprühvorgang auf der Deckschicht in Teilschichten aufgetragen bis eine Schichtdicke der wirkstoffhaltigen Schicht von 300 µm erreicht worden war.
c) Es wurde eine Dispersion aus 6 g Polyacrylsäure vernetzt mit Divinylglycol (Polycarbophil®) in 494 g Wasser hergestellt. Auch diese Dispersion wurde mit Hilfe der vorstehend angegebenen Apparatur in einem mehrmaligen Sprühvorgang, bei dem jeweils die Teilschichten erzeugt wurden, bis eine Schichtdicke der Haftschicht von 50 µm erreicht wurden war.
Die auf diese Weise hergestellte Darreichungsform war leicht zu handhaben und leicht auf die menschliche Haut sowie auf die menschlichen Schleimhäute, zum Beispiel auf die buccale Schleimhaut, aufzubringen.

### Beispiel 5 (nicht von schutzbereich der Anspruche ein geschlossen)

Zur Herstellung der filmförmigen Darreichungsform wurde eine Lösung von 10 g Hydroxypropylmethylcellulose, 3,125 g Glycerin, 0,5 g des Wirkstoffes Prednisolon, und 486,375 g Wasser sowie eine Lösung aus 2,5 g Tannin in 497,5 g Wasser hergestellt. Mit Hilfe der in DE 101 46 251 beschriebenen Apparatur wurden diese beiden Lösungen jeweils mit einer Düse gleichzeitig auf eine Glasplatte aufgesprüht, bei 80 °C getrocknet und der Sprühvorgang nach Bildung der jeweiligen Teilschicht mehrmals wiederholt, bis eine Schichtdicke von 300 µm erreicht worden war. Die auf diese Weise hergestellte Darreichungsform war leicht zu handhaben und leicht auf die menschliche Haut sowie auf die menschlichen Schleimhäute, zum Beispiel auf die buccale Schleimhaut, aufzubringen.

### Beispiel 6 (nicht vom schutzbereich der Anspruche ein geschlossen)

Zur Herstellung der filmförmigen Darreichungsform wurde eine Lösung von 10 g Hydroxypropylmethylcellulose, 2,5 g Glycerin, 0,5 g des Wirkstoffes Prednisolon, und 487 g Wasser sowie eine Lösung aus 2,5 g Tannin in 497,5 g Wasser hergestellt. Mit Hilfe der in DE 101 46 251 beschriebenen Apparatur wurden diese beiden Lösungen jeweils mit einer Düse gleichzeitig auf eine Glasplatte aufgesprüht, bei 80 °C getrocknet und der Sprühvorgang nach Bildung der jeweiligen Teilschicht mehrmals wiederholt, bis eine Schichtdicke von 300 µm erreicht worden war.

Die auf diese Weise hergestellte Darreichungsform war leicht zu handhaben und leicht auf die menschliche Haut sowie auf die menschlichen Schleimhäute, zum Beispiel auf die buccale Schleimhaut, aufzubringen.

### Beispiel 7

Zur Herstellung der filmförmigen Darreichungsform wurde eine Lösung von 10 g Hydroxypropylmethylcellulose, 6,25 g Glycerin, 0,5 g des Wirkstoffes Prednisolon, und 483,25 g Wasser sowie eine Lösung aus 2,5 g Tannin in 497,5 g Wasser hergestellt. Mit Hilfe der in DE 101 46 251 beschriebenen Apparatur wurden diese beiden Lösungen jeweils mit einer Düse gleichzeitig auf eine Glasplatte aufgesprüht, bei 80 °C getrocknet und der Sprühvorgang nach Bildung der jeweiligen Teilschicht mehrmals wiederholt, bis eine Schichtdicke von 300 µm erreicht worden war.
Die auf diese Weise hergestellte Darreichungsform war leicht zu handhaben und leicht auf die menschliche Haut sowie auf die menschlichen Schleimhäute, zum Beispiel auf die buccale Schleimhaut, aufzubringen.

### Vergleichsbeispiel 1

Zur Herstellung der filmförmigen Darreichungsform ohne Weichmacher wurde eine Lösung von 10 g Hydroxypropylmethylcellulose, 0,5 g des Wirkstoffes Prednisolon, und 489,5 g Wasser sowie eine Lösung aus 2,5 g Tannin in 497,5 g Wasser hergestellt. Mit Hilfe der in DE 101 46 251 beschriebenen Apparatur wurden diese beiden Lösungen jeweils mit einer Düse gleichzeitig auf eine Glasplatte aufgesprüht, bei 80 °C getrocknet und der Sprühvorgang nach Bildung der jeweiligen Teilschicht mehrmals wiederholt, bis eine Schichtdicke von 300 µm erreicht worden war.

Die auf diese Weise hergestellte Darreichungsform war schwer auf die menschliche Haut sowie auf die menschlichen Schleimhäute, zum Beispiel auf die buccale Schleimhaut, aufzubringen.

### Vergleichsbeispiel 2

Zur Herstellung der filmförmigen Darreichungsform wurde eine Lösung von 10 g Hydroxypropylmethylcellulose, 3,125 g Polyethylenglycol, 0,5 g des Wirkstoffes Prednisolon, und 486,375 g Wasser sowie eine Lösung aus 2,5 g Tannin in 497,5 g Wasser hergestellt. Mit Hilfe der in DE 101 46 251 beschriebenen Apparatur wurden diese beiden Lösungen jeweils mit einer Düse gleichzeitig auf eine Glasplatte aufgesprüht, bei 80 °C getrocknet und der Sprühvorgang nach Bildung der jeweiligen Teilschicht mehrmals wiederholt, bis eine Schichtdicke von 300 µm erreicht worden war.
Die auf diese Weise hergestellte Darreichungsform leichter auf die menschliche Haut sowie auf die menschlichen Schleimhäute, zum Beispiel auf die buccale Schleimhaut, aufzubringen, als die Darreichungsform aus dem Vergleichsbeispiel 1 (kein Weichmacher), anderseits schwerer aufzubringen als die Darreichungsform aus dem Beispiel 5 mit 25 Gew.-%, bezogen auf die Gesamtmenge vemetzter Hydroxypropylmethylcellulose, Glycerin.

### Vergleichsbeispiel 3

Zur Herstellung der filmförmigen Darreichungsform wurde eine Lösung von 10 g Hydroxypropylmethylcellulose, 3,125 g Sorbitol, 0,5 g des Wirkstoffes Prednisolon, und 486,375 g Wasser sowie eine Lösung aus 2,5 g Tannin in 497,5 g Wasser hergestellt. Mit Hilfe der in DE 101 46 251 beschriebenen Apparatur wurden diese beiden Lösungen jeweils mit einer Düse gleichzeitig auf eine Glasplatte aufgesprüht, bei 80 °C getrocknet und der Sprühvorgang nach Bildung der jeweiligen Teilschicht mehrmals wiederholt, bis eine Schichtdicke von 300 µm erreicht worden war.
Die auf diese Weise hergestellte Darreichungsform leichter auf die menschliche Haut sowie auf die menschlichen Schleimhäute, zum Beispiel auf die buccale Schleimhaut, aufzubringen" als die Darreichungsform aus dem Vergleichsbeispiel 1 (kein Weichmacher), anderseits schwerer aufzubringen als die Darreichungsform aus dem Beispiel 5 mit 25 Gew.-%, bezogen auf die Gesamtmenge vernetzter Hydroxypropylmethylcellulose, Glycerin.

### Vergleichsbeispiel 4

Zur Herstellung der filmförmigen Darreichungsform wurde eine Lösung von 10 g Hydroxypropylmethylcellulose, 2,5 g Triethylcitrat, 0,5 g des Wirkstoffes Prednisolon, und 487 g Wasser sowie eine Lösung aus 2,5 g Tannin in 497,5 g Wasser hergestellt. Mit Hilfe der in DE 101 46 251 beschriebenen Apparatur wurden diese beiden Lösungen jeweils mit einer Düse gleichzeitig auf eine Glasplatte aufgesprüht, bei 80 °C getrocknet und der Sprühvorgang nach Bildung der jeweiligen Teilschicht mehrmals wiederholt, bis eine Schichtdicke von 300 µm erreicht worden war.
Die auf diese Weise hergestellte Darreichungsform leichter auf die menschliche Haut sowie auf die menschlichen Schleimhäute, zum Beispiel auf die buccale Schleimhaut, aufzubringen" als die Darreichungsform aus dem Vergleichsbeispiel 1 (kein Weichmacher), anderseits schwerer aufzubringen als die Darreichungsform aus dem Beispiel 3 mit 20 Gew.-%, bezogen auf die Gesamtmenge vernetzter Hydroxypropylmethylcellulose, Glycerin.

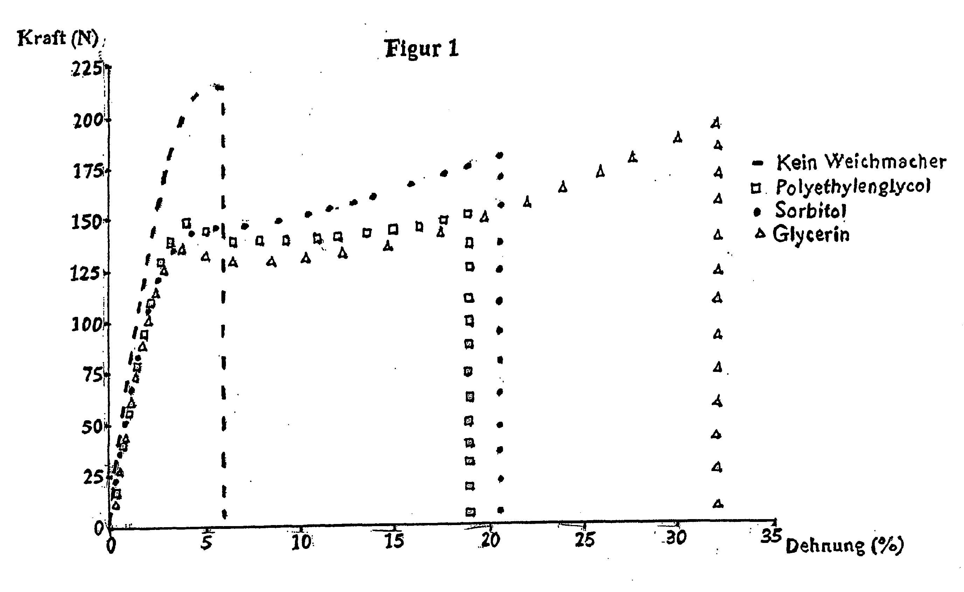

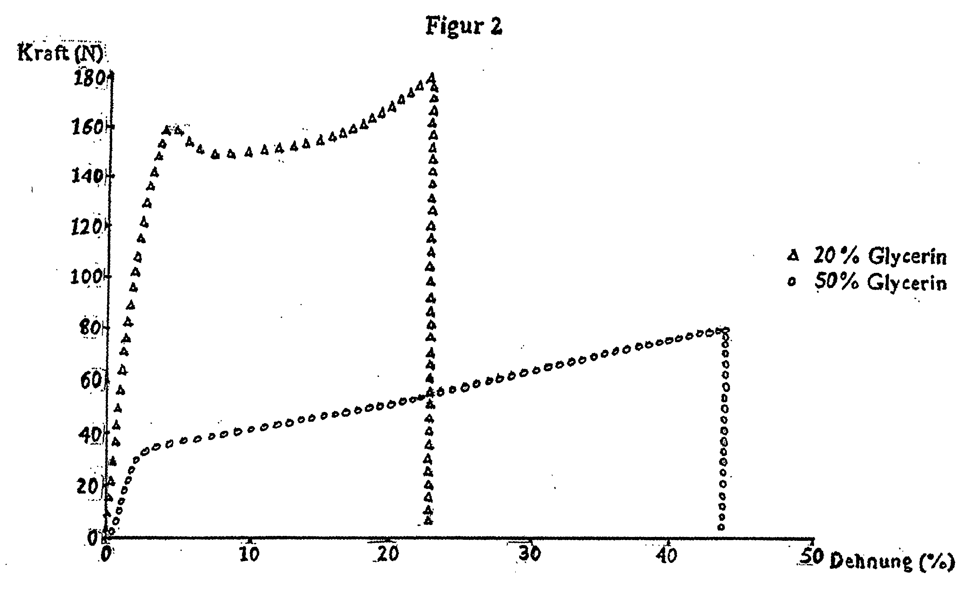

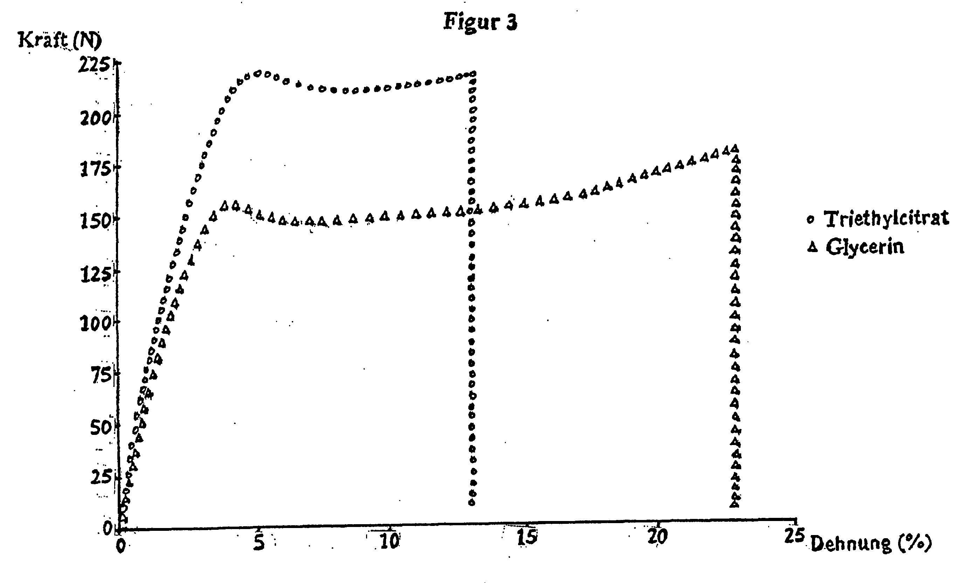

## Patentansprüche

1. Filmförmige Darreichungsform zur oberflächlichen Verabreichung wenigstens eines Wirkstoffes und/oder Nährstoffes an ein Lebewesen umfassend wenigstens eine wirkstoffhaltige und/oder nährstoffhaltige Schicht basierend auf in-situ vernetzten hydrophilen Polymeren, die 30 bis 60 Gew. % Glycerin, bezogen auf die Gesamtmenge der vernetzten hydrophilen Polymere, als Weichmacher enthält und wobei die Darreichungsform eine Reißfestigkeit von mindestens 40 N aufweist.

2. Darreichungsform nach Anspruch 1, **dadurch gekennzeichnet, dass** als hydrophiles Polymeres Hydroxypropylmethylcellulose verwendet wurde.

3. Darreichungsform nach einem der Ansprüche 1 bis 2, **dadurch gekennzeichnet, dass** das hydrophile Polymer mit Tannin und/oder einer vernetzten, gegebenenfalls teilneutralisierten Polyacrylsäure vernetzt wurden.

4. Darreichungsform nach einem der Ansprüche 1 bis 3, **dadurch gekennzeichnet, dass** die wirkstoffhaltige und/oder nährstoffhaltige Schicht wenigstens einen pharmazeutischen Wirkstoff oder einen Nährstoff enthält.

5. Darreichungsform nach Anspruch 4, **dadurch gekennzeichnet, dass** der pharmazeutische Wirkstoff ein Wirkstoff aus der Gruppe der Analgetica, Antiallergica, Antibiotica, Antiemetica, Antiseptica, Antihistaminica, Antihypertonia, Appetitzügler, Cardiaca, Chemotherapeutica, Enzyme, Hormone, Immunmodulatoren, Impfstoffen, Lokalanästhetica, Psychopharmaka, Spasmolytica, Virustatica, Vitamine und Zytostatica ist.

6. Darreichungsform nach Anspruch 4, **dadurch gekennzeichnet, dass** der Nährstoff ein Düngemittel ist.

7. Darreichungsform nach einem der Ansprüche 1 bis 3, **dadurch gekennzeichnet, dass** sie einschichtig oder mehrschichtig ist.

8. Darreichungsform nach Anspruch 7, **dadurch gekennzeichnet, dass** sie wenigstens eine wirkstoffhaltige und/oder nährstoffhaltige Schicht, eine Haftschicht und/oder eine Deckschicht aufweist.

9. Darreichungsform nach Anspruch 8, **dadurch gekennzeichnet, dass** wenigstens eine wirkstoffhaltige und/oder nährstoffhaltige Schicht einen Konzentration-Gradienten des Wirkstoffs und/oder des Nährstoffs aufweist.

10. Darreichungsform nach Anspruch 8, **dadurch gekennzeichnet, dass** die Deckschicht für den Wirkstoff undurchlässig ist.

11. Darreichungsform nach einem der Ansprüche 1 bis 10, **dadurch gekennzeichnet, dass** sie vor der Applikation mit einer Schutzschicht bedeckt ist.

12. Darreichungsform nach einem der Ansprüche 1 bis 11, **dadurch gekennzeichnet, dass** das Lebewesen ein Mensch oder ein Tier, bevorzugt ein Mensch ist.

13. Darreichungsform nach einem der Ansprüche 1 bis 12, **dadurch gekennzeichnet, dass** die oberflächliche Verabreichung eine transmukosale oder transdermale, bevorzugt eine transmukosale Verabreichung ist.

## Claims

1. Dosage form in film form for surface administration of at least one active ingredient and/or nutrient to a living creature comprising at least one active ingredient-containing and/or nutrient-containing layer based on in-situ crosslinked hydrophilic polymers which comprises from 30 to 60% by weight of glycerol, based on the total amount of crosslinked hydrophilic polymers, as plasticizer and the dosage form having a tear strength of at least 40 N.

2. Dosage form according to Claim 1, **characterized in that** hydroxypropylmethylcellulose was used as hydrophilic polymer.

3. Dosage form according to any of Claims 1 to 2, **characterized in that** the hydrophilic polymer has been crosslinked with tannin and/or a crosslinked, optionally partially neutralized polyacrylic acid.

4. Dosage form according to any of Claims 1 to 3, **characterized in that** the active ingredient-containing and/or nutrient-containing layer comprises at least one active pharmaceutical ingredient or one nutrient.

5. Dosage form according to Claim 4, **characterized in that** the active pharmaceutical ingredient is an active ingredient from the group of analgesics, antiallergics, antibiotics, antiemetics, antiseptics, antihistamines, antihypertensives, appetite suppressants, cardiac remedies, chemotherapeutic agents, enzymes, hormones, immunomodulators, inoculations, local anesthetics, psychoactive drugs, spasmolytics, virustatics, vitamins and cytostatics.

6. Dosage form according to Claim 4, **characterized in that** the nutrient is a fertilizer.

7. Dosage form according to any of Claims 1 to 3, **characterized in that** it has one or more layers.

8. Dosage form according to Claim 7, **characterized in that** it has at least one active ingredient-containing and/or nutrient-containing layer, one adhesive layer and/or one covering layer.

9. Dosage form according to Claim 8, **characterized in that** at least one active ingredient-containing and/or nutrient-containing layer has a concentration gradient of the active ingredient and/or of the nutrient.

10. Dosage form according to Claim 8, **characterized in that** the covering layer is impermeable for the active ingredient.

11. Dosage form according to any of Claims 1 to 10, **characterized in that** it is covered by a protective layer before application.

12. Dosage form according to any of Claims 1 to 11, **characterized in that** the living creature is a human or an animal, preferably a human.

13. Dosage form according to any of Claims 1 to 12, **characterized in that** the surface administration is a transmucosal or transdermal, preferably a transmucosal administration.

## Revendications

1. Forme d'administration sous forme de film pour une administration superficielle d'au moins un agent actif et/ou d'un nutriment à un être vivant, comprenant au moins une couche contenant l'agent actif et/ou le nutriment à base de polymères hydrophiles réticulés in situ, contenant de 30 à 60% en poids de glycérine, par rapport à la quantité totale de polymères hydrophiles réticulés, comme plastifiant, ladite forme d'administration présentant une résistance à la rupture d'au moins 40 N.

2. Forme d'administration selon la revendication 1, **caractérisée en ce que** de l'hydroxypropylmethylcellulose est utilisée comme polymère hydrophile.

3. Forme d'administration selon l'une des revendications 1 à 2, **caractérisée en ce que** le polymère hydrophile est réticulé avec des tannins et/ou un acide polyacrylique réticulé, le cas échéant partiellement neutralisé.

4. Forme d'administration selon l'une des revendications 1 à 3, **caractérisée en ce que** la couche contenant l'agent actif et/ou le nutriment contient au moins un agent actif pharmaceutique ou un nutriment.

5. Forme d'administration selon la revendication 4, **caractérisée en ce que** l'agent actif pharmaceutique est un agent actif choisi parmi les analgésiques, les antiallergiques, les antibiotiques, les antiémétiques, les antiseptiques, les antihistaminiques, les antihypertoniques, les anorexigènes, les remèdes cardiaques, les chimiothérapeutiques, les enzymes, les hormones, les modulateurs d'immunité, les vaccins, les anesthésiques locaux, les psychotropes, les spasmolytiques, les virustatiques, les vitamines et les cytostatiques.

6. Forme d'administration selon la revendication 4, **caractérisée en ce que** le nutriment est un fertilisant.

7. Forme d'administration selon l'une des revendications 1 à 3, **caractérisée en ce qu'**elle est monocouche ou multicouche.

8. Forme d'administration selon la revendication 7, **caractérisée en qu'**elle présente au moins une couche d'agent actif et/ou de nutriment, une sous-couche et/ou couche de couverture.

9. Forme d'administration selon la revendication 8, **caractérisée en qu'**au moins une couche d'agent actif et/ou de nutriment présente un gradient de concentration d'agent actif et/ou de nutriment.

10. Forme d'administration selon la revendication 8, **caractérisée en ce que** la couche de couverture est imperméable à l'agent actif.

11. Forme d'administration selon l'une des revendications 1 à 10, **caractérisée en ce qu'**elle est recouverte avant l'application par une couche de protection.

12. Forme d'administration selon l'une des revendications 1 à 11, **caractérisée en ce que** l'être vivant est un être humain ou un animal, de préférence un être humain.

13. Forme d'administration selon l'une des revendications 1 à 12, **caractérisée en ce que** l'administration superficielle est une administration transmucosale, ou transdermique, de préférence transmucosale.
